# EUROPEAN PATENT SPECIFICATION

(11) **EP 3 131 457 B1**
(45) Date of publication and mention of the grant of the patent: **26.05.2021**
(21) Application number: 15825412.8
(22) Date of filing: 14.07.2015
(51) Int. Cl.: A61B 3/10, A61F 9/008, A61B 34/00, A61B 5/00, A61B 3/113, A61F 9/007, A61B 90/00, A61B 17/00, A61B 34/20

(54) **OPTICAL COHERENCE TOMOGRAPHY-AUGMENTED SURGICAL INSTRUMENTS AND SYSTEMS FOR CORRECTING UNDESIRED MOVEMENT OF SURGICAL INSTRUMENTS**
DURCH OPTISCHE KOHÄRENZTOMOGRAPHIE ERWEITERTE CHIRURGISCHE INSTRUMENTE SOWIE SYSTEME ZUR KORREKTUR VON UNERWÜNSCHTER BEWEGUNG VON CHIRURGISCHEN INSTRUMENTEN
INSTRUMENTS CHIRURGICAUX AUGMENTÉS PAR TOMOGRAPHIE À COHÉRENCE OPTIQUE ET SYSTÈMES POUR CORRIGER DES MOUVEMENTS INTEMPESTIFS DES INSTRUMENTS CHIRURGICAUX

(30) Priority: 25.07.2014 US 201414341752
(43) Date of publication of application: 22.02.2017
(73) Proprietor: Alcon Inc., 1701 Fribourg (CH)
(72) Inventor: YU, Lingfeng, Lake Forest, California 92630 (US); REN, Hugang, Lake Forest, California 92630 (US)
(74) Representative: Hanna Moore + Curley
(86) International application number: PCT/US2015/040360
(87) International publication number: WO 2016/014289

(56) References cited:
- WO-A1-2014/043517
- US-A1- 2006 055 938
- US-A1- 2009 262 359
- US-A1- 2013 123 759
- US-A1- 2014 028 997
- US-A1- 2014 078 512
- US-A1- 2014 160 484
- US-B1- 6 564 087
- ZHANG KANG, KANG JIN U: "Common-path low-coherence interferometry fiber-optic sensor guided microincision", JOURNAL OF BIOMEDICAL OPTICS, vol. 16, no. 9, 095003, 1 September 2011 (2011-09-01), pages 095003-1-095003-4, XP009500760, ISSN: 1083-3668, DOI: 10.1117/1.3622492
- SUNGWOOK YANG ET AL: "Optical coherence tomography scanning with a handheld vitreoretinal micromanipulator", ENGINEERING IN MEDICINE AND BIOLOGY SOCIETY (EMBC), 2013 35TH ANNUAL INTERNATIONAL CONFERENCE OF THE IEEE, IEEE, 28 August 2012 (2012-08-28), pages 948-951, XP032463074, ISSN: 1557-170X, DOI: 10.1109/EMBC.2012.6346089

## Description

### BACKGROUND

### Field of the Disclosure

The present disclosure relates to optical coherence tomography (OCT)-augmented surgical instruments and to systems and methods for correcting for undesired movement of surgical instruments using OCT.

### Description of the Related Art

Surgery often involves precise removal of tissue or placement of incisions. In microsurgery, in particular, accurate positioning in all three dimensions as well as precise motion control is critical to avoid unintended effects, such as an inability to complete the surgery or even damage. This is especially true for ophthalmic surgeries. For example, in vitreoretinal surgeries, tool-tip positioning accuracy of around 10 µm is desired. Hand tremor is a common problem in surgeries and is difficult to avoid, yet it can regularly cause movements of a much as 50 µm. This is well outside of the desired range for vitreoretinal surgery and other microsurgeries and reduces the quality of these surgeries.

Other surgeries, such as treatment of retinal vein occlusion, are impossible to perform because movement of the surgical instruments cannot be properly controlled. Retinal vein occlusion affect 1.6% of people aged 49 and older and can be treated surgically, but currently the procedure is considered too risky to perform.

Prior approaches to combat undesired movement such as accidental movement in microsurgical procedures actively compensate for tremors. For example, one approach places a magnetometer-aided accelerometer on the surgical instrument to detect and compensate for tremors. Other approaches using proximal-end accelerometers to sense distal-end motion require complex data filtering and processing and add significant weight to the surgical instrument, which tends to cause more tremors.

Microsurgical instruments, systems, and methods that can compensate for tremors or other undesired movement in a more practical fashion are still needed.

Reference is made to the cited documents US 2013/123759, WO 2014/043517, US 2014/160484 and ZHANG KANG, KANG JIN U: "Common-path low-coherence interferometry fiber-optic sensor guided microincision", JOURNAL OF BIOMEDICAL OPTICS, val. 16, no. 9, 095003, 1 September 2011 (2011-09-01), pages 095003-1-095003-4, ISSN: 1083-3668, DOI: 10.1117/1.3622492, which provide an indication of the state of the art. Reference is also made to the publication as follows: Sungwook Yang Et Al: "Optical coherence tomography scanning with a handheld vitreoretinal micromanipulator", Engineering in Medicine and Biology Society (EMBC), 2013 35th Annual International Conference of the IEEE, IEEE, 28 August 2012 (2012-08-28), pages 948-951, XP032463074, ISSN: 1557-170X, DOI: 10.11 09/EMBC.2012.6346089 which discusses an arrangement in which motion compensation is provided based on detection of the motion of IR LEDs affixed to the instrument.

### SUMMARY

The scope of the invention is defined by the claims. Accordingly, there is provided an optical coherence tomography (OCT)-augmented surgical instrument (200) as defined in claim 1 and an optical coherence tomography (OCT) system as defined in claim 2. Further features are provided in the dependent claims.

In an arrangement of the specification, not claimed, there is provided an OCT system containing an OCT source coupled via an OCT transmission medium to a beam splitter coupled via one OCT path to a reference arm; and via a second OCT transmission medium to an OCT-augmented surgical instrument, as defined in claim 1.

In another arrangement, not claimed, there is provided to an OCT-augmented surgical instrument containing an OCT transmission medium, an OCT focusing element, an actuator, and a surgical component. The actuator is able to cause corrective movement of the surgical instrument in response to an OCT beam that travels through the OCT transmission medium and OCT focusing element.

In another arrangement of the specification, not claimed, there is provided a method of correcting undesired movement of a surgical instrument by sending an OCT beam from an OCT source via an OCT transmission medium to a beam splitter, which splits the beam into an OCT beam that travels to and is reflected by a reference arm and an OCT beam that travels to and is reflected by a tissue being operated on by the surgical instrument, detecting an interference pattern for the OCT beams reflected by the reference arm and tissue, determining whether an undesired movement occurred and an appropriate corrective movement based on the interference pattern, and causing the appropriate corrective movement in the surgical instrument using an actuator located in the surgical instrument.

### BRIEF DESCRIPTION OF THE DRAWINGS

For a more complete understanding of the present invention and its features and advantages, reference is now made to the following description, taken in conjunction with the accompanying drawings, which are not drawn to scale, and in which:
FIG. 1 is an OCT system containing an OCT-augmented surgical instrument;
FIG. 2 is an OCT-augmented surgical instrument;
FIG. 3 is another embodiment of an OCT-augmented surgical instrument;
FIG. 4 is a beam-splitting and focusing unit in an OCT-augmented surgical instrument;
FIG. 5 is another embodiment of a beam-splitting and focusing unit in an OCT-augmented surgical instrument;
FIG. 6 is a coupler in an OCT-augmented surgical instrument; and
FIG 7 is diagram depicting the relationship of measured vectors in space using an OCT system containing an OCT-augmented surgical instrument.

### DESCRIPTION OF PARTICULAR EMBODIMENT(S)

In the following description, details are set forth by way of example to facilitate discussion of the disclosed subject matter. It should be apparent to a person of ordinary skill in the field, however, that the disclosed embodiments are exemplary and not exhaustive of all possible embodiments.

As used herein, a reference numeral followed by a letter refers to a specific instance of an element and the numeral only form of the reference numeral refers to the collective element. Thus, for example, device '12a' refers to an instance of a device class, which may be referred to collectively as devices '12' and any one of which may be referred to generically as a device '12'.

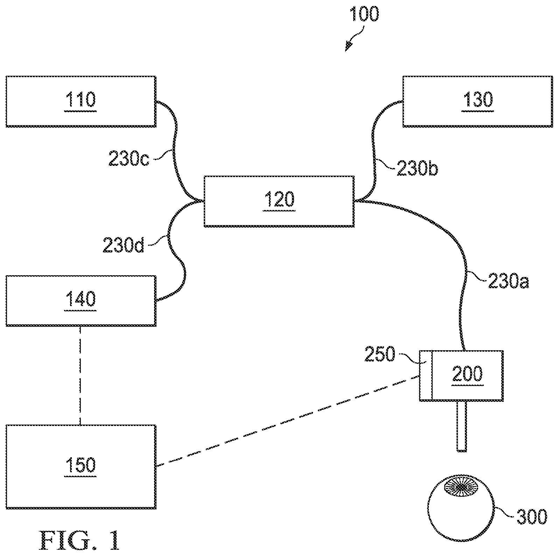

Referring now to the drawings, FIG. 1 is an OCT system 100 with OCT-augmented surgical instrument 200. Optical coherence tomography (OCT) is an interferometric analysis technique for structural examination of a sample material, such as a tissue that is at least partially reflective to light. It can also be used for functional examination of a sample material, such as the motion and velocity of the sample material or blood flow of the tissue. In OCT, light in the form on an OCT beam is used to measure distances and depth profiles based on optical interference that arises between a reference beam and a sample beam that interacts with the sample material, such as a biological tissue. In some embodiments, the OCT beam may be supplied in pulses, sweeping wavelengths or a broad band light.

OCT system 100 may make measurements of both the relative motion and velocity between surgical instrument 200 and tissue 300 (represented as an eye in this example diagram).

OCT system 100 additionally includes OCT source 110, which produces an OCT beam (not shown) that travels through OCT transmission medium 230c to beam splitter 120 where it is split so that a portion of the beam travels through OCT transmission medium 230b to reference arm 130 and a portion of the beam travels through OCT transmission medium 230a to surgical instrument 200. After hitting reference arm 130 or tissue 300, the OCT beams travel back through OCT transmission mediums 230b and 230a, respectively, to beam splitter 120, where they are directed via OCT transmission medium 230d to detector 140. Detector 140 sends a signal to computer 150, which includes a processor able to determine the relative motion and velocity of surgical instrument 200 with respect to tissue 300 based on the signal received from detector 140. Computer 150 determines if corrective movement of surgical instrument 200 is needed and, if so, sends a signal to an actuator 250 in surgical instrument 200. Actuator 250 responds to the signal and causes corrective movement of surgical instrument 200 in real-time.

In some embodiments, OCT transmission medium 230 is an optical fiber.

In the embodiment shown in FIG. 1, reference arm 130 is located close to tissue 300 in terms of optical delay and is in a pre-determined position that is an acceptable distance from the OCT source 110. The OCT beam from tissue 300 traveling back through surgical instrument 200 to detector 140, interferes with the OCT beam from reference arm 130 and generates an interference pattern, As a result, the motion characteristics (such as the gap, displacement and velocity) of the surgical instrument 200 relative to tissue 300 can be determined.

In one embodiment, reference arm 130 includes a mirror to reflect the OCT beam.

In one embodiment, detector 140 is a spectrometer. In another embodiment, detector 140 includes a photodiode or similar device that generates an electrical signal indicative of incident light intensity at detector 140.

Detector 140 may output an electrical signal to computer 150. In such an embodiment, computer 150 may include circuitry for signal conditioning, demodulation, digitization, and digital signal processing. In another embodiment, detector 140 outputs a wireless signal to computer 150.

In one embodiment, computer 150 additionally includes memory media, which store instructions (i.e., executable code) that are executable by the processor having access to the memory media. The processor may execute instructions that cause actuator 250 in surgical instrument 200 to activate and which control the parameters of such activation to allow compensation for undesired movement of surgical instrument 200. For the purposes of this disclosure, the memory media may include non-transitory computer-readable media that stores data and instructions for at least a period of time. The memory media may comprise persistent and volatile media, fixed and removable media, and magnetic and semiconductor media. The memory media may include, without limitation, storage media such as a direct access storage device (e.g., a hard disk drive or floppy disk), a sequential access storage device (e.g., a tape disk drive), compact disk (CD), random access memory (RAM), read-only memory (ROM), CD-ROM, digital versatile disc (DVD), electrically erasable programmable read-only memory (EEPROM), flash memory, non-transitory media, and various combinations of the foregoing.

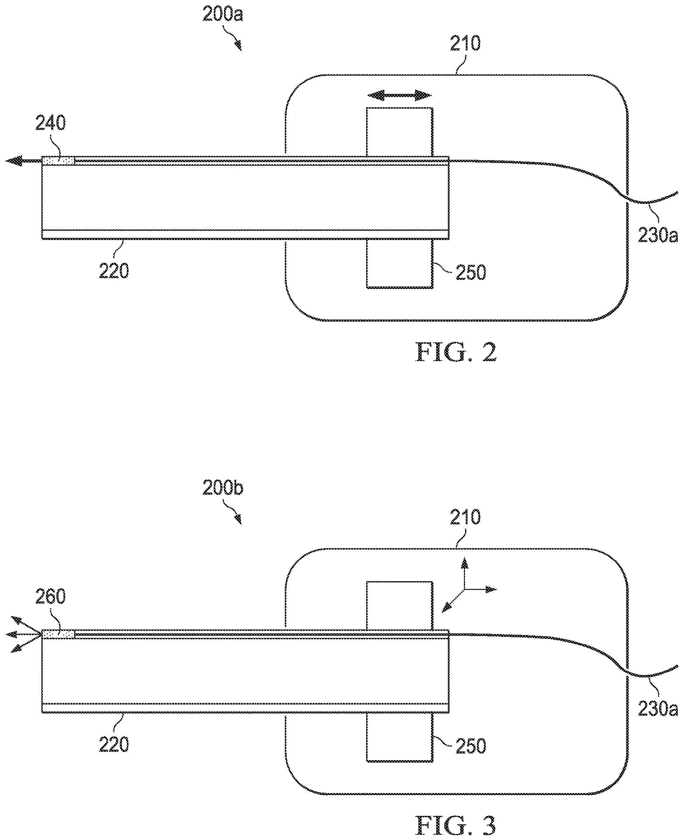

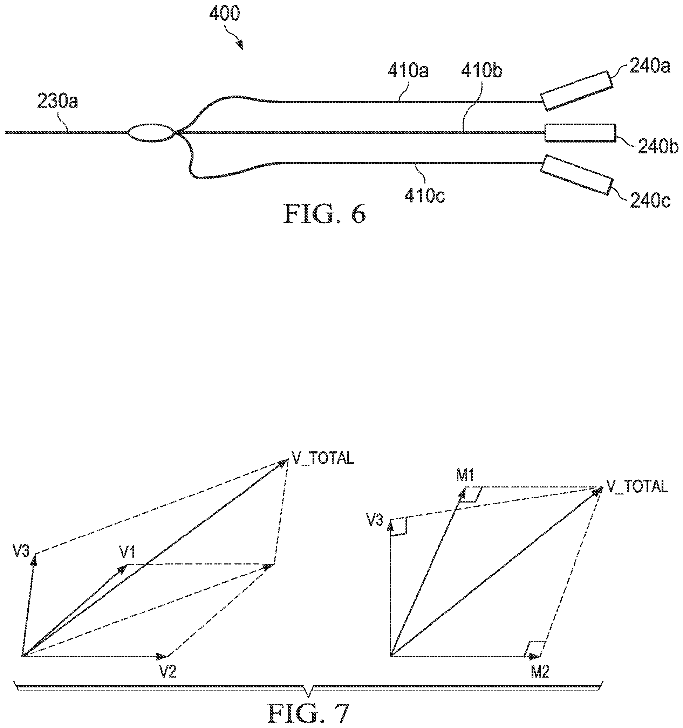

FIG. 2 depicts surgical instrument 200a not forming part of the invention, which may be used in an OCT system, and which includes handle 210 and cannula 220. In some embodiments, cannula 220 may be replaced with a different surgical component that performs a surgical operation. OCT transmission medium 230a travels through handle 210 into cannula 220, where it terminates with focusing element (e.g., lens, curved mirror) 240. Handle 210 also contains actuator 250, which is operable to receive a signal from a computer (not shown) and to cause movement of surgical instrument 200a in response to the signal. Because only one OCT beam travels through focusing element 240, the instrument in FIG. 2 provides one-dimensional OCT measurements. A surgical instrument may include multiple OCT transmission mediums and focusing lenses similar to those depicted in FIG. 2. These multiple OCT transmission mediums may be OCT fibers coupled via a coupler as shown in FIG. 6.

In the example shown, in which surgical instrument 200 contains cannula 220, actuator 250 moves cannula 220 in and out of handle 210 to compensate for undesired movement. For example, if the OCT system determines that cannula 220 is too close to the tissue (not shown), actuator 250 moves cannula 220 into handle 210 to compensate.

In some embodiments, actuator 250 moves cannula 220 or another surgical component at a speed that matches the speed of the undesired movement of surgical instrument 200. Actuator 250 may also move cannula 220 or another surgical component in a direction opposite the direction of the undesired movement, or a component direction of the undesired movement.

Actuator 250 may be any surgical actuator capable of moving surgical instrument 200 in real-time in response to undesired movement. In some embodiments, actuator 250 may constitute a small proportion of the weight of surgical instrument 200 in order to avoid introducing additional tremor. For example, actuator 250 may be less than 25% of the weight of surgical instrument 200. In one embodiment, actuator 250 is a piezoelectric actuator. In another embodiment, actuator 250 is a voice coil actuator. In still another embodiment, actuator 250 is an electromagnetic actuator. In another embodiment, actuator 250 is an ultrasonic actuator. Actuator 250 may be capable of movement in only one direction as shown in FIG. 2 or in two, three, or more directions as shown in FIG. 3, which illustrates movement in three directions. In embodiments where actuator 250 is capable of movement in two or more directions, actuator 250 may contain multiple components or sub-actuators, each capable of movement in one direction.

FIG. 3 depicts an alternative surgical instrument 200b, which, instead of focusing element 240, contains beam splitting and focusing unit 260, which splits the OCT beam traveling along OCT transmission medium 230a into two or more separate OCT beams focused in two or more directions. In one embodiment according to the invention, the beam is split into three or more OCT beams focused in three or more directions. The multiple split beams produced by beam splitting and focusing unit 260 have slightly different optical path length delay, so that OCT information from different beams will be separated in depth in corresponding OCT images. Using these different images, the multi-dimensional displacement and velocity of the tissue relative to surgical instrument 200, and vice versa, is calculated.

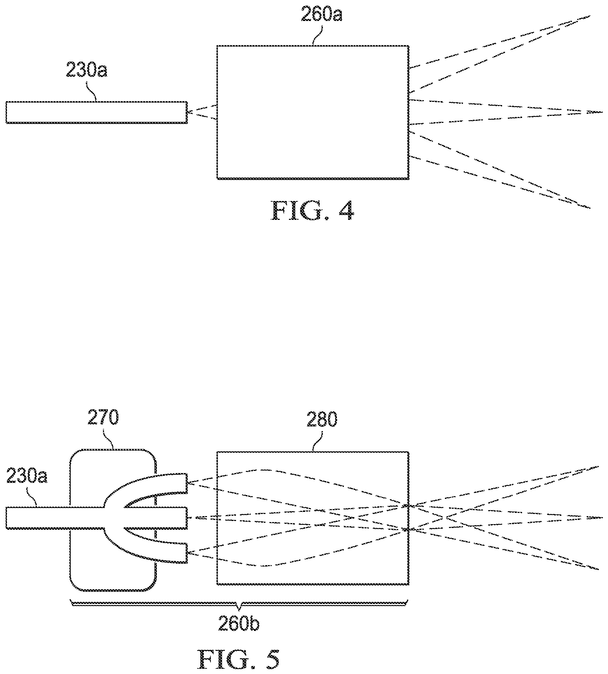

FIG. 4 depicts a unified beam splitting and focusing unit 260a. The beam splitting and focusing unit both splits the OCT beam into multiple beams and focuses those beams on the tissue (not shown).

FIG. 5 depicts an alternative beam splitting and focusing unit 260b according to the invention, which contains a separate beam splitting unit 270 and beam focusing unit 280. Beam splitting unit 270, in some embodiments, is a fiber splitter or a multi-cladding fiber. Beam focusing unit 280, in some embodiments is a graded index (GRIN) lens. In other embodiments, beam splitting and focusing unit 260b is a multiple faceted ball, such as a sapphire ball.

FIG. 6 depicts a coupler 400 for splitting the OCT beam into multiple OCT fibers 410a, 410b, and 410c, which terminate in focusing elements 240a, 240b, and 240c, respectively. The multiple OCT fibers and focusing elements may be located in surgical instrument 200 in a manner similar to that depicted in FIG. 2. OCT fibers 410 may be of slightly different lengths to cause different optical path delays.

In another embodiment, the detector is able to detect polarization of light and the OCT beam through the surgical instrument is split by polarization into different orientations. This also allows multi-dimensional measurements of motion and velocity of the tissue and surgical instrument with respect to one another.

In still another embodiment, not shown, the OCT beam may be split into different spectral bands in different orientations using one or more dispersive optical elements or dichroic beam splitting optical elements. In this embodiment, the detector is able to detect the different spectral bands. This embodiment also allows multi-dimensional measurements of motion and velocity of the tissue and surgical instrument with respect to one another.

In a specific example embodiment, supplying three separate OCT beams to a tissue, FIG. 7 depicts the relationship of the beam vectors, V1, V2, and V3, in space, which represents the orientation of each beam, as well as a combined vector, V_total. The beam vectors may be displacement vectors or velocity vectors representative of a tissue with respect to a surgical instrument, and vice versa. The orientations of the beam vectors to the surgical instrument are known because they are based on instrument design or prior calibration. Note that the beam vectors, V1, V2, and V3 are the projections of the overall motion vector V_total on each beam directions. The OCT system measures the magnitude of each beam vector and calculates the overall motion vector V_total. In order to compensate the undesired motion, the surgical instrument uses multiple actuators for active motion compensation. For three-dimensional motion compensation, normally three actuators are required. Note that the orientation of the actuator motion vectors M1, M2, M3 are considered known parameters as well, but they may be not overlapping with those of the OCT beam vectors V1, V2, V3. The projection of the overall motion vector V_total onto those actuator motion directions, M1, M2, M3 can be easily calculated, and used to guide the actual motion compensation of the surgical tool.

In one embodiment, the OCT system corrects for undesired movement in the surgical instrument by sending an OCT beam from the OCT source through an OCT transmission medium to the beam splitter, which splits the OCT beam to a beam that travels to the reference arm and a beam that travels to the surgical instrument. The OCT beam in the reference arm is reflected back and travels to a detector via an OCT transmission medium, for example through the beam splitter, which may recombine it with a beam from the surgical instrument. The OCT beam in the surgical instrument is reflected back by the tissue and also travels to a detector via an OCT transmission medium, for example through the beam splitter, which may recombine it with a beam from the reference arm. The detector detects the reflected OCT beam, either as a combined beam or as components from the reference arm and surgical instrument. The detector typically detects an interference pattern, which is altered if the surgical instrument experiences a pre-determined degree of undesired movement. The detector sends an electrical or wireless signal to the computer, which then uses its processor to determine whether undesired movement has occurred and the appropriate corrective movement. The computer then sends an electrical or wireless signal to the actuator in the surgical instrument to cause corrective movement. This corrective movement may occur in real-time. For example, it may occur in less than a millisecond.

OCT-augmented surgical instruments of the types described above may be used in microsurgeries, such as vitreoretinal surgeries, including intraoccular cannulation, injection of anticoagulants to treat occlusions, and atriovenous sheathotomy, otorhinolaryngological surgeries, neurological surgeries, laproscopic surgery, prostate surgery, and microvascular surgeries. Positioning of the surgical instrument tip may be controlled to an accuracy of 10 µm or less.

## Claims

1. An optical coherence tomography (OCT)-augmented surgical instrument (200) comprising:
an OCT transmission medium (230a);
an OCT focusing element (240);
an actuator (250) capable of moving the surgical element (200) in two or more directions; and
a surgical component adapted to perform a surgical operation;
wherein the actuator (250) is operable to cause corrective movement of the surgical instrument (200) in response to an OCT beam that travels through the OCT transmission medium (230a) and the OCT focusing element (240);
**characterized in that**
the OCT-augmented surgical instrument (200) further comprises a splitting element (260) that splits the OCT beam into three OCT beams of different orientations.

2. An optical coherence tomography (OCT) system (100) comprising:
an OCT source (110);
a beam splitter (120) coupled to the OCT source (110) via a first OCT transmission medium (230c);
a reference arm (130) coupled to the beam splitter (120) via a second OCT transmission medium (230b);
an OCT-augmented surgical instrument (200) according to claim 1 coupled via the OCT transmission medium thereof, which defines a third OCT transmission medium (230a), to the beam splitter (120);
a detector (140) coupled via a fourth OCT transmission medium (230d) to the beam splitter, wherein the detector (140) is adapted to receive an OCT beam containing a component from the reference arm and a component from the OCT-augmented surgical instrument;
the system (100) further comprising a computer (150) electrically or wirelessly coupled to the detector and the actuator,
wherein an undesired movement of the surgical instrument (200) results in a change in an interference pattern detected by the detector (140), which is communicated to the computer,
wherein the computer (150) is configured to determine, as undesired motion of the OCT-augmented surgical instrument, a relative motion and velocity of surgical instrument (200) with respect to tissue (300) based on the signal received from detector (140);
wherein the computer (150) is further configured to send an electrical or wireless signal to the actuator (250) to cause a corrective movement of the surgical instrument (200) in at least two directions to compensate said undesired motion of the OCT-augmented surgical instrument;
wherein the computer (150) is configured to determine said relative motion and velocity between the surgical instrument (200) and a tissue according to three beam vectors (V1, V2, V3) of the three OCT beams, wherein the three beam vectors (V1, V2 and V3) are the projections of the overall motion vector (V_total) on each beam direction;
wherein the OCT system measures the magnitude of each of three beam vectors and calculates the overall motion vector (V_total) based on the three beam vectors.

3. The OCT system of Claim 2, wherein the multiple OCT beams have different optical path delays.

4. The OCT system of claim 2 wherein the multiple OCT beams have different polarizations.

5. The OCT system of claim 2 wherein the multiple OCT beams have different spectral bands.

6. The OCT system of Claim 2, wherein the splitting element (260, 270) and the OCT focusing element (240, 280) are comprised in a beam splitting and focusing unit (260a,b).

7. The OCT system of claim 6, wherein the beam splitting and focusing unit comprises a separate beam splitting unit (270) and a separate beam focusing unit (280).

8. The OCT system of claim 2, wherein the splitting element comprises a coupler (240) and at least three OCT fibers (410a,b,c).

9. The OCT system of Claim 2, wherein the splitting element (260) splits the OCT beam into at least three OCT beams.

10. The OCT system of Claim 2, wherein the focusing element (240) comprises a focusing lens or wherein the focusing element comprises a curved mirror.

11. The OCT system of Claim 2, wherein the surgical component comprises a cannula (220).

## Patentansprüche

1. Durch optische Kohärenztomographie (OCT) erweitertes chirurgisches Instrument (200), umfassend:
ein OCT-Übertragungsmedium (230a):
ein OCT-Fokussierelement (240);
einen Aktor (250), der in der Lage ist, das chirurgische Element (200) in zwei oder mehr Richtungen zu bewegen; und
eine chirurgische Komponente, die vorgesehen ist, um eine chirurgische Operation durchzuführen;
wobei der Aktor (250) funktional ist, um korrigierende Bewegung des chirurgischen Instruments (200) in Reaktion auf einen OCT-Strahl zu bewirken, der sich durch das OCT-Übertragungsmedium (230a) und das OCT-Fokussierelement (240) hindurch bewegt;
**dadurch gekennzeichnet, dass**
das OCT-erweiterte chirurgische Instrument (200) des Weiteren ein Teilerelement (260) umfasst, welches den OCT-Strahl in drei OCT-Strahle mit unterschiedlichen Orientierungen teilt.

2. Optisches Kohärenztomographie- (OCT)-System (100), umfassend:
eine OCT-Quelle (110);
einen Strahlteiler (120), der über ein erstes OCT-Übertragungsmedium (230c) an die OCT-Quelle (110) gekoppelt ist;
einen Referenzarm (130), der über ein zweites OCT-Übertragungsmedium (230b) an den Strahlteiler (120) gekoppelt ist;
ein OCT-erweitertes chirurgisches Instrument (200) nach Anspruch 1, das über dessen OCT-Übertragungsmedium, das ein drittes OCT-Übertragungsmedium (230a) definiert, an den Strahlteiler (120) gekoppelt ist;
einen Detektor (140), der über ein viertes OCT-Übertragungsmedium (230d) an den Strahlteiler gekoppelt ist, wobei der Detektor (140) vorgesehen ist, um einen OCT-Strahl zu empfangen, der eine Komponente von dem Referenzarm und eine Komponente von dem OCT-erweiterten chirurgischen Instrument enthält;
wobei das System (100) des Weiteren einen Computer (150) umfasst, der elektrisch oder drahtlos an den Detektor und den Aktor gekoppelt ist,
wobei eine unerwünschte Bewegung des chirurgischen Instruments (200) zu einer Änderung in einem Interferenzmuster führt, das durch den Detektor (140) detektiert wird, welche an den Computer kommuniziert wird,
wobei der Computer (150) konfiguriert ist, um als unerwünschte Bewegung des OCT-erweiterten chirurgischen Instruments eine relative Bewegung und Geschwindigkeit des chirurgischen Instruments (200) in Bezug zu Gewebe (300) basierend auf dem Signal zu bestimmen, welches von dem Detektor (140) empfangen wurde; wobei der Computer (150) des Weiteren konfiguriert ist, um ein elektrisches oder drahtloses Signal an den Aktor (250) zu senden, um eine korrigierende Bewegung des chirurgischen Instruments (200) in mindestens zwei Richtungen zu bewirken, um die unerwünschte Bewegung des OCT-erweiterten chirurgischen Instruments zu kompensieren;
wobei der Computer (150) konfiguriert ist, um die relative Bewegung und Geschwindigkeit zwischen dem chirurgischen Instrument (200) und einem Gewebe gemäß drei Strahlvektoren (V1, V2, V3) der drei OCT-Strahle zu bestimmen, wobei die drei Strahlvektoren (V1, V2 und V3) die Projektionen des Gesamtbewegungsvektors (V_gesamt) auf jede Strahlrichtung sind;
wobei das OCT-System den Betrag von jedem der drei Strahlvektoren misst und den Gesamtbewegungsvektor (V_gesamt) basierend auf den drei Strahlvektoren berechnet.

3. OCT-System nach Anspruch 2, wobei die mehreren OCT-Strahle unterschiedliche Verzögerungen des optischen Pfads aufweisen.

4. OCT-System nach Anspruch 2, wobei die mehreren OCT-Strahle unterschiedliche Polarisierungen aufweisen.

5. OCT-System nach Anspruch 2, wobei die mehreren OCT-Strahle unterschiedliche Spektralbanden aufweisen.

6. OCT-System nach Anspruch 2, wobei das Teilerelement (260, 270) und das OCT-Fokussierelement (240, 280) in einer Strahlteiler- und -fokussiereinheit (260a, b) enthalten sind.

7. OCT-System nach Anspruch 6, wobei die Strahlteiler- und -fokussiereinheit eine separate Strahlteilereinheit (270) und eine separate Strahlfokussiereinheit (280) umfasst.

8. OCT-System nach Anspruch 2, wobei das Teilerelement einen Koppler (240) und mindestens drei OCT-Fasern (410a, b, c) umfasst.

9. OCT-System nach Anspruch 2, wobei das Teilerelement (260) den OCT-Strahl in mindestens drei OCT-Strahle teilt.

10. OCT-System nach Anspruch 2, wobei das Fokussierelement (240) eine Fokussierlinse umfasst, oder wobei das Fokussierelement einen gekrümmten Spiegel umfasst.

11. OCT-System nach Anspruch 2, wobei die chirurgische Komponente eine Kanüle (220) umfasst.

## Revendications

1. Instrument chirurgical augmenté par tomographie par cohérence optique (TCO) (200) comprenant :
un milieu de transmission TCO (230a) ;
un élément de mise au point TCO (240) ;
un actionneur (250) susceptible de déplacer l'élément chirurgical (200) dans deux directions ou plus ; et
un composant chirurgical conçu pour réaliser une opération chirurgicale ;
dans lequel l'actionneur (250) est exploitable pour provoquer un mouvement correctif de l'instrument chirurgical (200) en réponse à un faisceau TCO qui se déplace à travers le milieu de transmission TCO (230a) et l'élément de mise au point TCO (240) ;
**caractérisé en ce que**
l'instrument chirurgical augmenté par TCO (200) comprend en outre un élément de division (260) qui divise le faisceau TCO en trois faisceaux TCO de différentes orientations.

2. Système de tomographie par cohérence optique (TCO) (100) comprenant :
une source TCO (110) ;
un diviseur de faisceau (120) couplé à la source TCO (110) par l'intermédiaire d'un premier milieu de transmission TCO (230c) ;
un bras de référence (130) couplé au diviseur de faisceau (120) par l'intermédiaire d'un deuxième milieu de transmission TCO (230b) ;
un instrument chirurgical augmenté par TCO (200) selon la revendication 1 couplé par l'intermédiaire du milieu de transmission TCO de celuici, qui définit un troisième milieu de transmission TCO (230a), au diviseur de faisceau (120) ;
un détecteur (140) couplé par l'intermédiaire d'un quatrième milieu de transmission TCO (230d) au diviseur de faisceau, le détecteur (140) étant conçu pour recevoir un faisceau TCO contenant une composante en provenance du bras de référence et une composante en provenance de l'instrument chirurgical augmenté par TCO ;
le système (100) comprenant en outre un ordinateur (150) couplé électriquement ou sans fil au détecteur et à l'actionneur,
dans lequel un mouvement intempestif de l'instrument chirurgical (200) entraîne un changement d'un motif d'interférence détecté par le détecteur (140), qui est communiqué à l'ordinateur,
dans lequel l'ordinateur (150) est configuré pour déterminer, en tant que mouvement intempestif de l'instrument chirurgical augmenté par TCO, un mouvement relatif et une vitesse relative de l'instrument chirurgical (200) par rapport au tissu (300) sur la base du signal reçu en provenance détecteur (140) ;
dans lequel l'ordinateur (150) est en outre configuré pour envoyer un signal électrique ou sans fil à l'actionneur (250) pour provoquer un mouvement correctif de l'instrument chirurgical (200) dans au moins deux directions pour compenser ledit mouvement intempestif de l'instrument chirurgical augmenté par TCO ;
dans lequel l'ordinateur (150) est configuré pour déterminer ledit mouvement relatif et ladite vitesse relative entre l'instrument chirurgical (200) et un tissu en fonction de trois vecteurs de faisceaux (V1, V2, V3) des trois faisceaux TCO, dans lequel les trois vecteurs de faisceaux (V1, V2, V3) sont les projections du vecteur de mouvement global (V_total) sur chaque direction de faisceau ;
dans lequel le système TCO mesure la grandeur de chacun des trois vecteurs de faisceaux et calcule le vecteur de mouvement global (V_total) sur la base des trois vecteurs de faisceaux.

3. Système TCO selon la revendication 2, dans lequel les multiples faisceaux TCO ont différents retards de chemin optique.

4. Système TCO selon la revendication 2 dans lequel les multiples faisceaux TCO ont différentes polarisations.

5. Système TCO selon la revendication 2 dans lequel les multiples faisceaux TCO ont différentes bandes spectrales.

6. Système TCO selon la revendication 2, dans lequel l'élément de division (260, 270) et l'élément de mise au point TCO (240, 280) sont compris dans une unité de division et de mise au point de faisceau (260a, b).

7. Système TCO selon la revendication 6, dans lequel l'unité de division et de mise au point de faisceau comprend une unité de division de faisceau (270) distincte et une unité de mise au point de faisceau (280) distincte.

8. Système TCO selon la revendication 2, dans lequel l'élément de division comprend un coupleur (240) et au moins trois fibres TCO (410a, b, c).

9. Système TCO selon la revendication 2, dans lequel l'élément de division (260) divise le faisceau TCO en au moins trois faisceaux TCO.

10. Système TCO selon la revendication 2, dans lequel l'élément de mise au point (240) comprend une lentille de mise au point ou dans lequel l'élément de mise au point comprend un miroir incurvé.

11. Système TCO selon la revendication 2, dans lequel le composant chirurgical comprend une canule (220).
